Europäisches Patentamt

European Patent Office

Office européen des brevets

(19)

(11) Veröffentlichungsnummer : **0 098 537**
**B1**

(12)

# EUROPÄISCHE PATENTSCHRIFT

(45) Veröffentlichungstag der Patentschrift :
**13.04.88**

(51) Int. Cl.⁴ : **A 61 M 15/00**

(21) Anmeldenummer : **83106399.5**

(22) Anmeldetag : **30.06.83**

(54) **Dampfinhaliergerät.**

(30) Priorität : **02.07.82 DE 3224849**

(43) Veröffentlichungstag der Anmeldung :
**18.01.84 Patentblatt 84/03**

(45) Bekanntmachung des Hinweises auf die Patenterteilung : **13.04.88 Patentblatt 88/15**

(84) Benannte Vertragsstaaten :
**AT BE CH DE FR GB IT LI LU NL SE**

(56) Entgegenhaltungen :
**EP-A- 0 047 072**
**AT-B- 210 560**
**BE-A- 692 821**
**DE-B- 2 331 525**
**DE-U- 1 615 022**
**DE-U- 7 921 306**
**FR-A- 788 612**
**FR-A- 1 338 422**

(73) Patentinhaber : **PLANTORGAN WERK Heinrich G.E. Christensen KG**
**Hornbusch 1**
**D-2903 Bad Zwischenahn (DE)**

(72) Erfinder : **Badewien, Reinhard**
**Königsmoorstrasse 41**
**D-2956 Moormerland/Veenhusen (DE)**

(74) Vertreter : **Kinzebach, Werner, Dr.**
**Patentanwälte Reitstötter, Kinzebach und Partner**
**Sternwartstrasse 4 Postfach 86 06 49**
**D-8000 München 86 (DE)**

## Beschreibung

Die Erfindung betrifft ein Dampfinhaliergerät mit einem Behälter für zu verdampfende Heilflüssigkeit und einem kegelförmigen Deckel, der an seinem spitzen Ende eine Mund und/oder Nase umfließende Atemmaske aufweist.

Ein derartiges Dampfinhaliergerät ist beispielsweise in der DE-U-79 21 306 beschrieben. Bei diesem bekannten Gerät befindet sich die Heilflüssigkeit in einem Innenbehälter, der von der Außenwand beabstandet ist, so daß die normalerweise heiße Heilflüssigkeit besser von der Umgebung isoliert ist.

Diese Heilflüssigkeit besteht üblicherweise aus heißem Wasser und einem zugesetzten Wirkstoff. Während der Behandlung werden Mund und Nase an die Atemmaske fest angedrückt, so daß die aus der heißen Heilflüssigkeit aufsteigende Dämpfe eingeatmet werden können.

Es hat sich gezeigt, daß dieses Gerät zwar äußerst einfach konstruiert ist, jedoch nicht immer optimal arbeitet, da die Verdampfung der Wirkstoffe in Abhängigkeit von der Temperatur der Heilflüssigkeit erfolgt und sehr schnell mit deren Abkühlung nachläßt. Zu Beginn der Behandlung ist die Temperatur der Heilflüssigkeit relativ hoch, so daß sich stark mit den Wirkstoffen vermischte Dämpfe bilden, die jedoch wegen ihrer ebenfalls noch hohen Eigentemperatur nur vorsichtig und langsam eingeatmet werden können. Dieser hohe Temperaturbereich, der die optimalen Bedingungen für die Verdampfung der Wirkstoffe und somit für den angestrebten Heilprozeß bietet, kann also garnicht ausgenützt werden.

Der mittlere Temperaturbereich, der sich während der Abkühlung der Heilflüssigkeit einstellt, ermöglicht ein angenehmes Einatmen der Dämpfe, jedoch sind dann die Heilwirkstoffe nur noch in geringerer Konzentration im einzuatmenden Dampf enthalten. Während der weiteren Behandlung wird dieser angenehme Temperaturbereich relativ schnell unterschritten und die Sättigung der inhalierten Dämpfe mit Heilwirkstoffen wird immer geringer. Letztlich wird die Behandlung oft vorzeitig abgebrochen, weil der Inhalierende feststellt, daß er keine heilenden Wirkstoffe mehr einatmet, obwohl diese zweifellos noch in der Heilflüssigkeit in genügender Menge vorhanden sind.

Es sind auch verschiedene Beatmungsgeräte im Stand der Technik beschrieben, beispielsweise in der DE-B-23 31 525. Da bei diesen Geräten der Patient beatmet wird, erfordert dies einen hohen konstruktiven Aufwand. Es handelt sich somit um keine Inhaliergeräte, bei denen der Patient aktiv atmen muß.

Der Erfindung liegt die Aufgabe zugrunde, ein Inhaliergerät der eingangs genannten Gattung derart auszubilden, daß eine Heilflüssigkeit unabhängig von der im Behälter befindlichen, zu verdampfenden Flüssigkeit in therapeutisch wirksamer Konzentration verdampft wird.

Gelöst wird diese Aufgabe durch ein Dampfinhaliergerät der eingangs genannten Gattung, das dadurch gekennzeichnet ist, daß im Deckel eine Zerstäubungsvorrichtung für eine zusätzliche Heilflüssigkeit angeordnet ist, die aus einem Einsatz, einem Teil und einem Justiereinsatz aufgebaut ist, wobei a) der Einsatz radial ausgerichtete Haltearme zur Abstützung im unteren Teil des Deckels aufweist und sich ein Kanal in einem dieser Haltearme befindet, der mit seinem nach außen weisenden Ende mit einer Verbindungsleitung zum Zuführen von Luft in Verbindung steht, und der Einsatz ferner einen auf den Haltearmen mittig angeordneten und senkrecht nach oben weisenden Distanzhalter und einen oberhalb des Distanzhalters angeordneten Behälter für die zusätzliche Heilflüssigkeit mit einem im wesentlichen mittig nach oben weisenden Rohrstutzen mit kegeliger Außenfläche, über deren im wesentlichen gesamte Länge mindestens eine Längsnut verläuft, aufweist, wobei sich in dem Rohrstutzen eine am oberen Ende des Rohrstutzens austretende Leitung befindet, die durch den Distanzhalter verläuft und mit dem Kanal verbunden ist, b) das Teil aus einem Mantel mit einer zum Rohrstutzen des Einsatzes komplementär konischen Bohrung besteht, die den Rohrstutzen derart umschließt, daß dessen Längsnut eine am Oberende des Rohrstutzens endende Leitung bildet, deren Ansaugöffnung am Fuß des Rohrstutzens liegt, und das Teil ferner ein mit dem Mantel verbundene, mit Bohrungen versehene Bodenplatte aufweist, welche in einen den Mantel umgebenden Außenmantel übergeht, und c) der Justiereinsatz in den Außenmanetel des Teils eingesetzt ist und in seinem Zentrum sich ein über radial ausgerichtete Arme gehaltenes, in vertikaler Richtung justierbares, gegenüber dem Oberende des Rohrstutzens angeordnetes Prallelement befindet, und daß der kegelförmige Deckel an seinem spitzen Ende einen in den Behälter ragenden Rohrstutzen besitzt.

In den Behälter der Zerstäubungsvorrichtung füllt man eine höher konzentrierte Lösung von Heilwirkstoffen, beispielsweise Öle, Salze usw. Das Prallelement wird vorteilhafterweise so geformt, daß die auftretenden Flüssigkeitströpfchen in den Behälter zurückfallen und nur der verdampfte Anteil an Heilflüssigkeit abgeatmet wird. Auf diese Weise sind eine vollständigere Nutzung des Wirkstoffs und ein stärkerer therapeutischer Effekt erzielbar.

In diesen Behälter kann man auch Zusatzwirkstoffe einfüllen. Diese werden dann über die Zerstäubungsvorrichtung als Aerosol dem aus der Heilflüssigkeit aufsteigenden Dampf beigemischt. Auf diese Weise wird eine relativ genaue Dosierung der Zusatzwirkstoffe erzielt, so daß eine optimale Heilwirkung mit dem erfindungsgemäßen Inhaliergerät erreicht werden kann.

Die Zerstäubungsdüse des erfindungsgemäßen Dampfinhaliergerätes besteht aus einem Rohrstutzen mit kegeliger Außenfläche, in die von oben nach unten verlaufend eine oder mehrere Längsnuten eingefräßt sind. Auf dem kegeligen Rohrstutzen sitzt ein Mantel mit komplementär kegeliger Bohrung, so daß die Nuten zwischen Mantel und Rohrstutzen Leitungen bilden, die vom Oberende des Rohrstutzen bis zu dessen Boden reichen, wobei der Mantel mit dem Oberende des Rohrstutzens abschließt, jedoch etwas kürzer als dieser ist. Der kegelige Rohrstutzen ragt vom Boden des Behälters mit der zusätzlichen Heilflüssigkeit hervor, so daß zwischen dem Mantel und dem Boden des Behälters die Einströmöffnungen für die Heilflüssigkeit entstehen.

Vorzugsweise ist der kegelförmige Rohrstutzen und der ihn umgebende Behälter für die zusätzliche Heilflüssigkeit einstückig mit dem Einsatz ausgebildet, der im Deckel eingesetzt werden kann. Darüber hinaus sind im Einsatz gasdichte Leitungen vorhanden, welche das Innere des Rohrstutzens mit der Verbindungsleitung verbinden.

Der auf dem Rohrstutzen aufgesetzte Mantel mit Innenkonus ist in einer größeren gelochten Platte in deren Mitte angeordnet, die einen Außenmantel trägt.

Im Außenmantel auf der größeren Platte ist ein Einsatz eingesetzt, in dem das Prallelement über Justiermittel gehalten ist. Diese ganze Anordnung, welche die Zerstäubungsvorrichtung bildet, ist reibschlüssig im Deckel des Dampfinhaliergerätes eingesetzt.

Vorzugsweise wird die Verdampfungsrate der Heilflüssigkeit dadurch vergrößert, daß man Ausströmöffnungen für Druckluft vorsieht, die im Behälter so angeordnet sind, daß sie unter dem Heilflüssigkeitsspiegel liegen. Die Druckluft wird beispielsweise mittels einer kleinen elektrischen Balgpumpe oder aber über einen einfachen Gummiball und eine dünne Schlauchleitung eingeblasen.

Die Einzelteile des Einsatzes sind vorteilhafterweise leicht auseinandernehmbar konzipiert. Auf diese Weise kann das Gerät leicht gereinigt werden, was im medizinischen Bereich immer besonders wichtig ist.

Im folgenden werden einige Ausführungsbeispiele anhand von Abbildungen näher erläutert, wobei

Figur 1 einen Längsschnitt durch eine bevorzugte Ausführungsform des Dampfinhaliergerätes,

Figur 2 einen Längsschnitt entlang der Linie VI-VI des Einsatzes nach Figur 2,

Figur 3 eine Draufsicht auf den Einsatz nach Figur 2,

Figur 4 einen Längsschnitt durch den Düsenmantel nach Figur 1 entlang der Linie IIX-IIX nach Figur 5,

Figur 5 einen Horizontalschnitt entlang der Linie IX-IX aus Figur 4,

Figur 6 einen Längsschnitt durch den Justiereinsatz nach Figur 1 entlang der Linie X-X aus Figur 7 und

Figur 7 eine Draufsicht auf den Justiereinsatz nach Figur 6 zeigt.

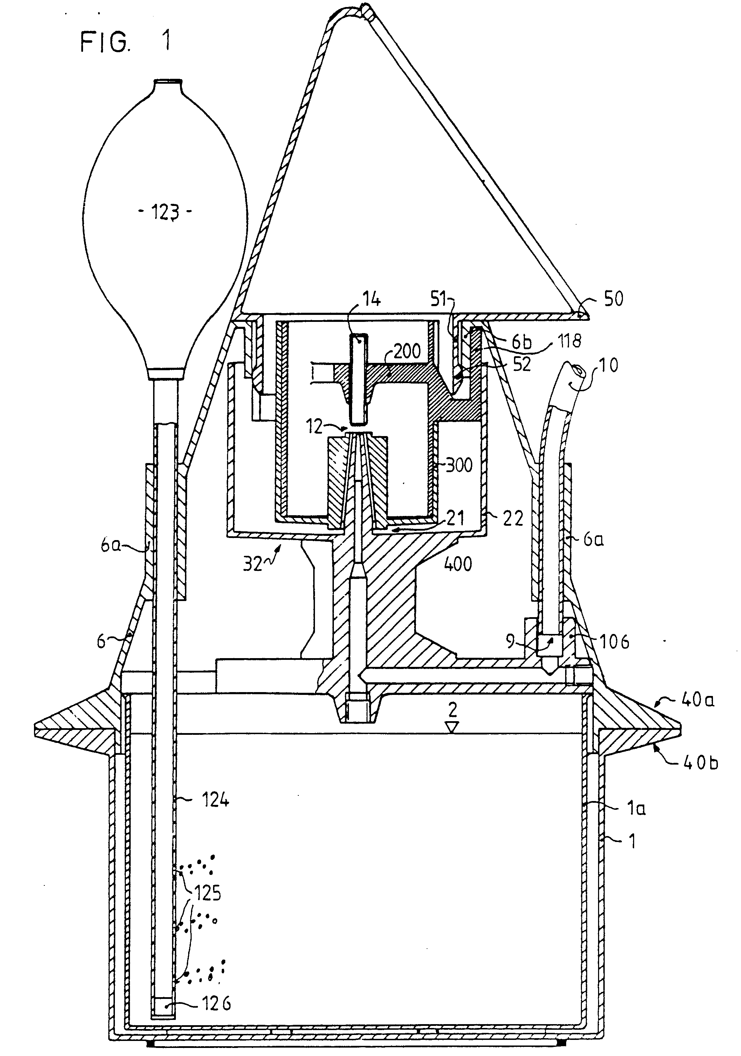

Bei der in den Figuren 1 bis 6 dargestellten bevorzugten Ausführungsform handelt es sich um ein Dampfinhaliergerät, das sowohl mit einer von Hand zu betätigenden Einblasvorrichtung, als auch mit einer Zerstäubungsvorrichtung zum Anschluß an eine Druckluftquelle (manuell oder motorisch betrieben) ausgelegt ist.

Nach Figur 1 weist das Dampfinhaliergerät einen im wesentlichen zylindrischen Behälter 1 auf, der an seinem Oberende mit zwei einander gegenüberstehend angeordneten Griffen 40b ausgerüstet ist. Der kegelförmige Deckel 6 weist an seinem Unterende einen ringförmigen Ansatz auf, der im Behälter 1 eng einrastet und ist ebenfalls mit Griffstücken 40a ausgerüstet, die auf den Griffstücken 40b fluchtend aufliegen, wenn der Deckel 6 auf dem Behälter 1 befestigt ist. Zur Befestigung dienen (hier nicht gezeigte) Verbindungsmittel zum Beispiel in Form eines Bajonettverschlusses, so daß der Deckel 6 auf dem Behälter 1 mit einer leichten Drehung aufgesetzt und so mit ihm fest verbunden werden kann. Im Behälter 1 ist ein Innenbehälter 1a angeordnet, dessen Außendurchmesser geringer als der Innendurchmesser des Behälters 1 ist, und der an seinem Boden Füßchen trägt. Auf diese Weise ist der Innenbehälter 1a durch eine Luftschicht vom Außenbehälter 1 thermisch isoliert. An seinem Oberende weist der kegelförmige Deckel 6 einen Abschlußrand auf, von dem ein Rohransatz 6b wieder in den Behälter hineinragt. Ebenfalls im Deckel 6 sind zwei beidseitig offene Rohrstutzen 6a angebracht, über welche die Luftzufuhr bewerkstelligt wird.

Die Atemmaske ist bei dieser bevorzugten Ausführungsform als gesonderte Maske 50 ausgebildet, die im wesentlichen eine Tetraederform aufweist (mit einer Spitze nach oben), wobei eine der Tetraederflächen vollständig offen ist. In der Tetraederfläche, die der Spitze gegenüberliegt, mündet ein Rohrstutzen 51, der an seinem der Maske abgewandten Ende eine ringförmige Wulst 52 aufweist. Mit dieser ringförmigen Wulst 52 inseriert die Maske 50 in den Rohrabschnitt 6b unter Reibschluß. Mittels dieser Anordnung kann die Maske 50 relativ zum Deckel 6 verdreht und etwas gekippt werden, was die Benutzung des Gerätes wesentlich erleichtert.

Durch eines der Rohre 6a im Deckel 6 ist die Ausblasleitung 124 eines von Hand zu betätigenden Blasebalges 123 so eingesetzt, daß das mit einem Stopfen 126 verschlossene Ende der Leitung 124 kurz über dem Boden des Innenbehälters 1a zu liegen kommt. In seinem unteren Bereich weist die Ausblasleitung 124 kleine, als Düsen wirkende Bohrungen 125 auf, die unterhalb des Flüssigkeitsspiegels 2 liegen.

Im Deckel 6 des Inhaliergerätes ist bei dieser bevorzugten Ausführungsform die Zerstäubungsvorrichtung 32 eingesetzt, in die Druckluft aus der Verbindungsleitung 10 gepreßt wird. Hierbei führt die Leitung 10 durch das zweite Rohr 6a im Deckel 6 und steckt mit ihrem Ende 9 im Luftzuführungsstutzen 106 der Zerstäubungsvorrichtung.

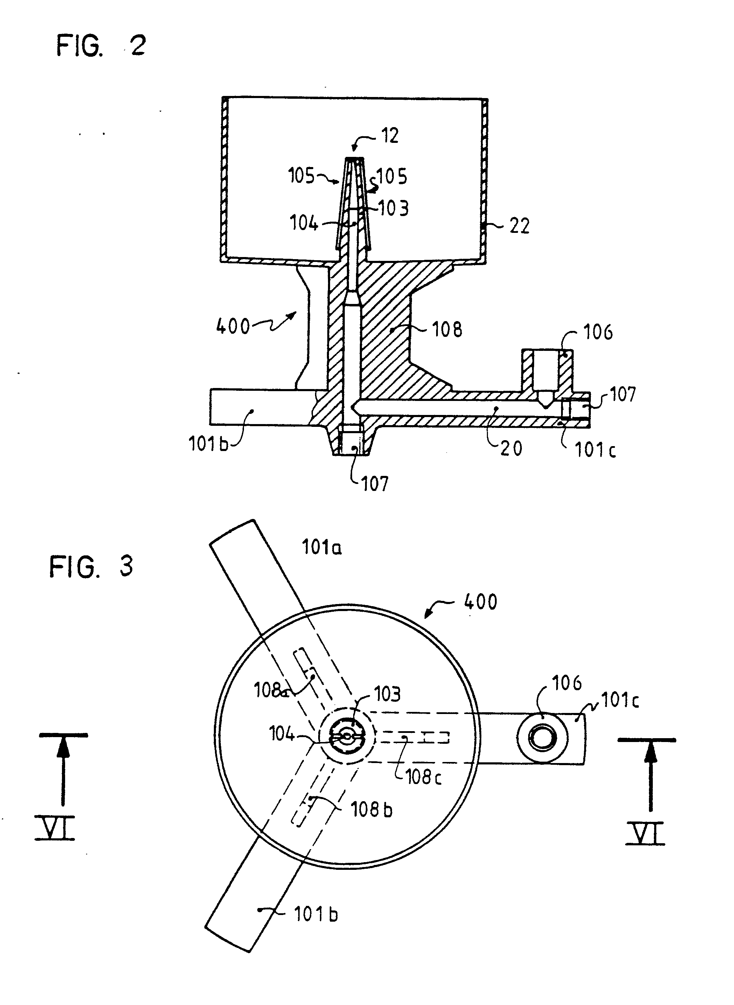

Die Zerstäubungsvorrichtung wird im folgenden anhand der Figuren 2 bis 7 näher beschrieben.

In den Figuren 2 und 3 ist eines der Teile der Zerstäubungsvorrichtung vergrößert dargestellt. Der im Deckel 6 angeordnete Einsatz 400 ist als einstückiges Kunststoff-Spritzgußteil ausgeführt und weist drei Arme 101a, 101b, 101c auf, wobei ein Arm (hier 101c) den Verbindungsstutzen 106 zum Anschluß an die Verbindungsleitung 10 (nicht gezeigt) trägt. Der Stutzen 106 steht über einen den Arm 101c durchdringenden Kanal 20 mit einem senkrechten Kanal 104 in Verbindung. Da im vorliegenden Fall die Kanäle gebohrt sind, sind entsprechende Abschlußstopfen 107 in den blinden Enden angebracht. Bei einer zweistückigen Fertigung, bei der zwei aufeinanderpassende Hälften mit eingesenkten Verbindungskanalhälften aufeinander geklebt werden, erübrigen sich diese Abschlußstopfen 107.

Senkrecht auf den drei Haltearmen 101 ist ein Distanzhalter 108 angeordnet, der den im wesentlichen zylindrischen Behälter 22 zur Aufnahme des pharmazeutischen Präparates trägt. Mittig zum Behälter 22 ist ein Rohrstutzen 103 vom Boden des Behälters nach oben ragend angebracht, dessen Inneres die Leitung 104 bildet, und der eine konische Außenfläche aufweist. In diese konischen Außenfläche sind zwei Nuten 105 eingesenkt.

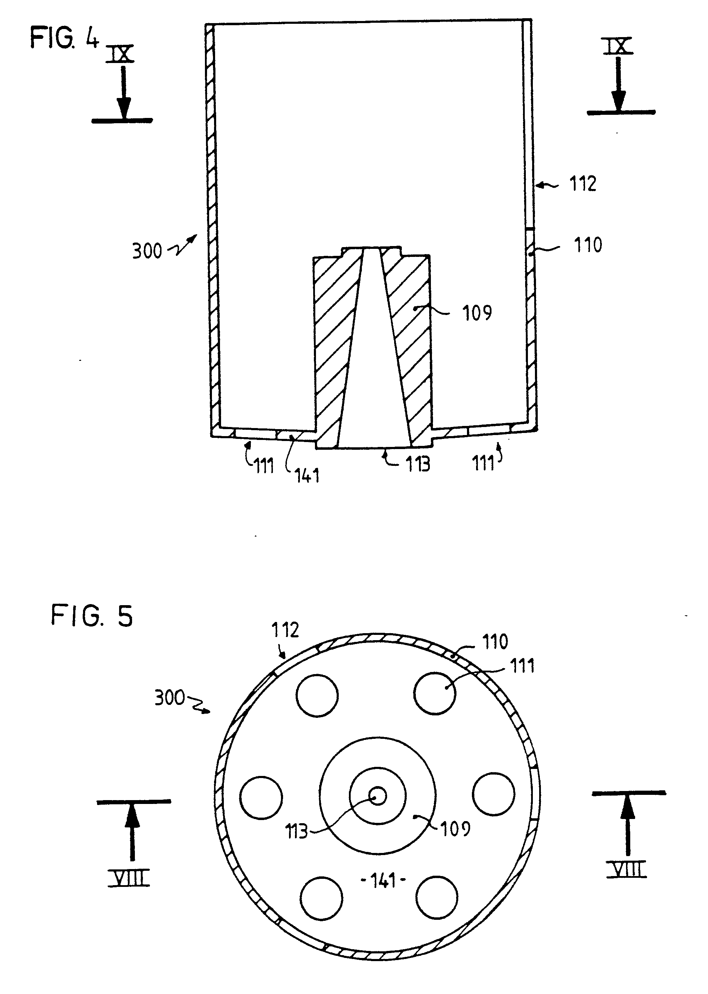

Wie dies aus Figur 5 hervorgeht, wird auf den mit dem Einsatz 400 einstückigen Rohrstutzen das Teil 300 aufgesetzt, das im folgenden anhand der Figuren 4 und 5 beschrieben wird. Das Teil 300 weist einen Mantel 109 auf, der eine im wesentlichen zylindrische Außenkontur, jedoch einem zum Rohrstutzen 103 komplementär konische Innenbohrung 113 aufweist. Die Länge des Mantels 109 ist hierbei so bemessen, daß das Oberende des Mantels nach Aufsetzen auf den Rohrstutzen 103 mit diesem bündig abschließt, während das Unterende des Mantels 109 etwas über dem Boden des Behälters 22 sitzt. Durch diese Dimensionierung ist gewährleistet, daß die Nuten 105 nach dem Aufsetzen des Mantels 109 auf den Rohrstutzen 103 Leitungen bilden, die sowohl oben, als auch unten, in der Nähe des Bodens des Behälters 22, offen sind. Sobald man den Behälter 22 mit einem zu zerstäubenden Mittel füllt und Druckluft in den Kanal 104 bringt, wird durch den über die Strömungsgeschwindigkeit der Druckluft am Austrittsende der Leitung 104 erzeugten Unterdruck Flüssigkeit aus dem Behälter 22 durch die Leitungen 105 angesaugt, mit der Druckluft verwirbelt und somit zerstäubt. Das Austrittsende der Leitung 104 bildet also zusammen mit den Nuten 105 die Zerstäubungsdüse 12.

Wie dies weiterhin aus den Figuren 4 und 5 hervorgeht, ist der Mantel 109 mit einer kreisförmigen Platte 141 verbunden, die in einen zylindrischen, den Mantel 109 umschließenden Außenmantel 110 übergeht. Durch Bohrungen 111 kann die zerstäubte Flüssigkeit, die nicht inhaliert wird, zurück in den Behälter 22 tropfen.

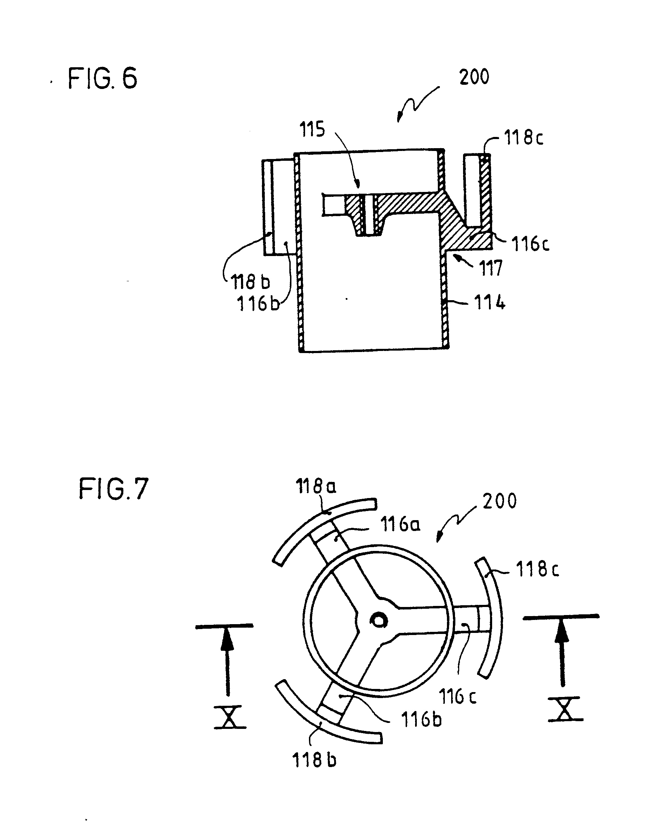

Wie dies aus Figur 1 hervorgeht, ist über der Zerstäubungsdüse 12 ein Prallelement 14 angeordnet, das in einem Justiereinsatz 200 gehalten ist, der im folgenden anhand der Figuren 6 und 7 näher beschrieben wird. Der Justiereinsatz 200 weist einen Außenmantel 114 auf, in dessen Zentrum ein Gewinde 115 über drei Arme 116a, 116b, 116c gehalten ist. In dieses Gewinde 115 wird das mit einem Außengewinde versehene Prallelement 14 eingeschraubt. Die Haltearme 116a, 116b, 116c ragen auch vom Außenmantel 114 nach außen und enden in zylinderausschnittsförmigen Fortsätzen 118a, 118b, 118c. Setzt man die Justiervorrichtung 200 für das Prallelement 14 in den Außenmantel 110 des Teils 300 (siehe Figur 1), so paßt der Justiereinsatz 200 gerade in das Element 300, wobei die Haltearme 116a, 116b, 116c in Schlitzen 112 (siehe Figuren 4 und 5) des Außenmantels 110 einrasten.

Wie dies aus Figur 1 hervorgeht, sind die Abschnitte 118a, 118b und 118c des Justiereinsatzes 200 so dimensioniert, daß sie den vom Oberrand des Deckels 6 nach innen ragenden Stutzen 6b gerade umschließen und so mit ihm in reibschlüssiger Verbindung stehen. Ebenso geht aus Figur 1 hervor, daß die Arme 101a, 101b, 101c des Einsatzes 400 eng an der Innenfläche des Deckels 6 dort anliegen, wo dieser in seinen unteren zylindrischen Abschnitt übergeht. Durch diese Anordnung der Zerstäubungsvorrichtung 32 ist gewährleistet, daß die Düse 12 mit dem Prallelement 14 immer richtig justiert ist, ohne daß hierfür besondere Einstellmaßnahmen nach jedem Zerlegen des Gerätes notwendig wären. Darüber hinaus ist durch die hier gezeigte Anordnung gewährleistet, daß man die drei Elemente 200, 300 und 400 jeweils einstückig aus Kunststoff fertigen kann, ohne daß ein größerer Aufwand durch Nachbearbeitung etc. notwendig wäre.

Aus Figur 1 geht auch hervor, daß der Boden des Behälters 22 leicht nach innen verlaufend abgesenkt ist, so daß die in den Behälter 22 eingebrachte zu vernebelnde Flüssigkeit gänzlich in die Ansaugöffnungen 21 gelangen kann.

Durch die mögliche Einstellung des Prallelementes 14 kann die Tröpfchengröße bei der Vernebelung je nach den Bedürfnissen eingestellt werden.

Die oben beschriebene Vorrichtung hat folgende Vorteile :

Wenn man ca. 80 °C warme Inhalationsflüssigkeit in den Innenbehälter 1a einbringt und lediglich den Balg 123 betätigt, so wird bei einer Zimmertemperatur von 21 °C am Mundstück eine Temperatur von 46 °C erreicht. Ohne Zuführung von zusätzlicher Luft wird jedoch lediglich eine Temperatur von 38 °C im austretenden Luft-Dampf-Gemisch erzielt.

Durch das mit erhöhter Temperatur ausströmende Luft-Dampf-Gemisch wird die Arzneimittelzubereitung zum Vernebeln im Behälter vorgewärmt. Das erhaltene Aerosol vermischt sich mit dem Luft-Dampf-Gemisch im Mundstück des Inhalators und wird dort bei gut konditionierter Temperatur eingeatmet, wobei der Behälter so dimensioniert werden muß, daß eine gute Vorwärmung über eine große Flüssigkeitsoberfläche erreicht wird.

Mit dem oben beschriebenen Gerät kann zusätzlich eine Vernebelung stattfinden.

Das beschriebene Gerät ist vielseitig anwendbar, man kann kalt vernebeln, warm vernebeln, kalt oder warm vernebeln mit zusätzlichem Einblasen von Luft (Sprudeleffekt), oder aber in der üblichen Art (ohne Sprudeln und Vernebeln) inhalieren.

**Patentansprüche**

1. Dampfinhaliergerät mit einem Behälter (1, 1a) für zu verdampfende Heilflüssigkeit und einem kegelförmigen Deckel (6), der an seinem spitzen Ende eine Mund und/oder Nase umschließende Atemmaske (50) aufweist, dadurch gekennzeichnet, daß im Deckel (6) eine Zerstäubungsvorrichtung (32) für eine zusätzliche Heilflüssigkeit angeordnet ist, die aus einem Einsatz (400), einem Teil (300) und einem Justiereinsatz (200) aufgebaut ist, wobei

a) der Einsatz (400) radial ausgerichtete Haltearme (101) zur Abstützung im unteren Teil des Deckels (6) aufweist und sich ein Kanal (20) in einem dieser Haltearme (101) befindet, der mit seinem nach außen weisenden Ende (9) mit einer Verbindungsleitung (10) zum Zuführen von Luft in Verbindung steht, und der Einsatz (400) ferner einen auf den Haltearmen (101) mittig angeordneten und senkrecht nach oben weisenden Distanzhalter (108) und einen oberhalb des Distanzhalters (108) angeordneten Behälter (22) für die zusätzliche Heilflussigkeit mit einem im wesentlichen mittig nach oben weisenden Rohrstutzen (103) mit kegeliger Außenfläche, über deren im wesentlichen gesamte Länge mindestens eine Längsnut (105) verläuft, aufweist, wobei sich in dem Rohrstutzen (103) eine am oberen Ende des Rohrstutzens (103) austretende Leitung (104) befindet, die durch den Distanzhalter (108) verläuft und mit dem Kanal (20) verbunden ist,

b) das Teil (300) aus einem Mantel (109) mit einer zum Rohrstutzen (103) des Einsatzes (400) komplementär konischen Bohrung besteht, die den Rohrstutzen (103) derart umschließt, daß dessen Längsnut (105) eine am Oberende des Rohrstutzens (103) endende Leitung bildet, deren Ansaugöffnung (21) am Fuß des Rohrstutzens (103) liegt, und das Teil (300) ferner ein mit dem Mantel (109) verbundene, mit Bohrungen (111) versehene Bodenplatte (141) aufweist, welche in einen den Mantel (109) umgebenden Außenmantel (110) übergeht, und

c) der Justiereinsatz (200) in den Außenmantel (110) des Teils (300) eingesetzt ist und in seinem Zentrum sich ein über radial ausgerichtete Arme (116) gehaltenes in vertikaler Richtung justierbares, gegenüber dem Oberende des Rohrstutzens (103) angeordnetes Prallelement (14) befindet, und daß der kegelförmige Deckel an seinem spitzen Ende einen in den Behälter (22) ragenden Rohrstutzen (6b) besitzt.

2. Dampfinhaliergerät nach Anspruch 1, dadurch gekennzeichnet, daß der Behälter (1, 1a), der Behälter (22), der Mantel (109) und der Außenmantel (110) zylindrische Außenkontur besitzen.

3. Dampfinhaliergerät nach Anspruch 1 oder 2, dadurch gekennzeichnet, daß der Einsatz (400) auf dem Oberrand des Innenbehälters (1a) aufsitzt, der im Behälter (1) mit Abstand zu dessen Innenwand gehalten ist.

4. Dampfinhaliergerät nach einem der vorhergehenden Ansprüche, dadurch gekennzeichnet, daß der Boden des Behälters (22) leicht nach innen verlaufend abgesenkt ist.

5. Dampfinhaliergerät nach einem der vorhergehenden Ansprüche, dadurch gekennzeichnet, daß der Justiereinsatz (200) Verbindungsmittel (118) aufweist, die mit dem vom Oberrand des kegelförmigen Deckels (6) nach innen ragenden Rohrstutzen (6b) in Reibschluß stehen und daß die Verbindungen zwischen dem Justiereinsatz (200) und dem Außenmantel (110) des Teiles (300) in Reibschluß ausgeführt sind.

6. Dampfinhaliergerät nach einem der vorhergehenden Ansprüche, dadurch gekennzeichnet, daß der Justiereinsatz (200), der Teil (300) und der Einsatz (400) jeweils einstückig sind.

7. Dampfinhaliergerät nach einem der vorhergehenden Ansprüche, dadurch gekennzeichnet, daß zur weiteren Steigerung der Verdampfungsrate Ausströmöffnungen (125) für Luft derart im Innenbehälter (1a) angeordnet sind, daß sie unter dem Heilflüssigkeitsspiegel (2) liegen und mit vorzugsweise manuell betätigbaren Pumpmitteln (123) in Verbindung stehen.

**Claims**

1. Vapour inhalation apparatus having a container (1, 1a) for a medicinal liquid which is to be vaporised and a conical cover (6) which has at its pointed end a breathing mask (50) fitting over the mouth and/or nose, characterised in that in the cover (6) is mounted an atomising device (32) for an additional medicinal liquid, which is made up of an insert (400), a part (300) and an adjusting insert (200), whilst

a) the insert (400) has radially aligned support arms (101) for resting in the lower part of the cover (6) and there is a channel (20) in one of these support arms (101) which communicates, at its outwardly pointing end (9), with a connecting conduit (10) for supplying air, and the insert (400) furthermore has a spacer (108) pointing vertically upwards and arranged centrally on the support arms (101) and a container (22) for the additional medicinal liquid arranged above the spacer (108), with a substantially central, upwardly pointing pipe connection (103) with a conical outer surface, at least one longitudinal groove (105) extending substantially over the entire length thereof, whilst in the pipe connection (103) there is a conduit (104) emerging at the upper end of the pipe connection (103), extending through the spacer (108) and connected to the channel (20),

b) the part (300) consists of a mantle (109) with a conical bore complementary to the pipe connection (103) of the insert (400), said bore enclosing the pipe connection (103) in such a way that the longitudinal groove (105) thereof forms a conduit ending at the upper end of the pipe connection (103), its intake opening (21) being at the base of the pipe connection (103), and the part (300) furthermore comprises a base plate (141) containing apertures (111) and connected to the mantle (109), said base plate merging into an outer casing (110) surrounding the mantle (109), and

c) the adjusting insert (200) is inserted in the outer casing (110) of the part (300) and in its centre is an impact element (14) which is vertically adjustable, held in place by radially aligned arms (116) and arranged opposite the upper end of the pipe connection (103), and in that the conical cover has, at its pointed end, a connection piece (6b) projecting into the container (22).

2. Vapour inhalation apparatus according to claim 1, characterised in that the container (1, 1a), the container (22), the mantle (109) and the outer casing (110) are cylindrical in their external contours.

3. Vapour inhalation apparatus according to claim 1 or 2, characterised in that the insert (400) rests on the upper edge of the inner container (1a) which is held in place in the container (1) spaced from the inner wall thereof.

4. Vapour inhalation apparatus according to one of the preceding claims, characterised in that the base of the container (22) sinks slightly inwardly.

5. Vapour inhalation apparatus according to one of the preceding claims, characterised in that the adjusting insert (200) has connecting means (118) which are frictionally connected to the connection piece (6b) projecting inwardly from the upper edge of the conical cover (6) and in that the connections between the adjusting insert (200) and the outer casing (110) of the part (300) are made by friction.

6. Vapour inhalation apparatus according to one of the preceding claims, characterised in that the adjusting insert (200), the part (300) and the insert (400) are each formed in one piece.

7. Vapour inhalation apparatus according to one of the preceding claims, characterised in that, in order further to increase the evaporation rate, outflow openings (125) for air are provided in the inner container (1a) in such a way that they are below the level (2) of the medicinal liquid and are connected to preferably manually operable pumping means (123).

**Revendications**

1. Inhalateur de vapeur, avec un récipient (1, 1a) pour un liquide thérapeutique à vaporiser et un couvercle tronconique (6) qui comporte à son extrémité du sommet un masque respiratoire (50) enfermant la bouche et/ou le nez, caractérisé par le fait que le couvercle (6) renferme, pour un liquide thérapeutique supplémentaire, un dispositif pulvérisateur (32) fait d'un corps insérable (400), d'une partie (300) et d'une armature d'ajustage (200), que :

a) le corps insérable (400) présente des bras de maintien (101) de direction radiale pour s'appuyer dans la partie inférieure du couvercle (6) et il se trouve dans l'un de ces bras de maintien (101) un canal (20) qui, par son extrémité (9) tournée vers l'extérieur, est relié à une conduite de jonction (10) pour l'amenée d'air, ledit corps insérable (400) comportant en outre, en position centrale sur les bras de maintien (101), un fût formant entretoise (108) dirigé verticalement vers le haut et, pour le liquide thérapeutique supplémentaire, au-dessus de ce fût entretoise (108) un réservoir (22) avec une tubulure essentiellement centrale (103) dirigée vers le haut et présentant une surface extérieure tronconique sur essentiellement toute la longueur de laquelle court au moins une rainure longitudinale (105), tandis qu'il se trouve dans ladite tubulure (103) un conduit (104) qui sort à l'extrémité supérieure de la tubulure (103) traverse le fût entretoise (108) et communique avec le canal (20) ;

b) la partie (300) se compose d'un manchon (109) muni d'un alésage de conicité complémentaire à celle de la tubulure (103) du corps insérable (400), lequel alésage entoure cette dernière (103) en sorte que la rainure longitudinale (105) de celle-ci forme un conduit qui se termine à l'extrémité supérieure de ladite tubulure (103) et dont l'orifice d'aspiration (21) est placé au pied de la tubulure (103) ladite partie (300) comportant en outre une plaque de fond (141), reliée au manchon (109) et munie de perforations (111), laquelle se poursuit en une enveloppe extérieure (110) entourant le manchon (109) ;

c) l'armature d'ajustage (200) est insérée dans l'enveloppe extérieure (110) de la partie (300) et il se trouve en son centre, en face de l'extrémité supérieure de la tubulure (103), un élément d'impact (14) tenu par des bras (116) de direction radiale et ajustable en direction verticale ; et que le couvercle tronconique possède à son extrémité du sommet un manchon (6b) pénétrant dans le réservoir (22).

2. Inhalateur de vapeur selon la revendication 1 caractérisé par le fait que le récipient (1, 1a), le réservoir (22), le manchon (109) et l'enveloppe extérieure (110) ont un contour extérieur cylindrique.

3. Inhalateur de vapeur selon la revendication 1 ou 2 caractérisé par le fait que le corps insérable (400) repose sur le bord supérieur du récipient intérieur (1a), maintenu dans le récipient (1) à une certaine distance de la paroi intérieure de ce dernier.

4. Inhalateur de vapeur selon l'une des revendications précédentes caractérisé par le fait que le fond du réservoir (22) est légèrement incliné vers l'intérieur.

5. Inhalateur de vapeur selon l'une des revendications précédentes caractérisé par le fait que l'armature d'ajustage (200) comporte des moyens de jonction (118) qui s'assemblent à friction au manchon (6b) faisant saillie intérieure à partir du bord supérieur du couvercle tronconique (6) et les liaisons entre l'armature d'ajustage (200) et l'enveloppe extérieure (110) de la partie (300) sont réalisées en assemblage par friction.

6. Inhalateur de vapeur selon l'une des revendications précédentes caractérisé par le fait que l'armature d'ajustage (200), la partie (300) et le corps insérable (400) sont réalisés chacun ou chacune en une seule pièce.

7. Inhalateur de vapeur selon l'une des revendications précédentes caractérisé par le fait que, pour accroître encore le taux de vaporisation, des ouvertures de dégagement d'air (125) sont disposées dans le récipient intérieur (1a) d'une façon telle qu'elles sont placées au-dessous du niveau de liquide thérapeutique (2) et reliées à des moyens de pompage (123) actionnables de préférence manuellement.

FIG. 1

0 098 537

FIG. 2

FIG. 3

FIG. 4

IX

IX

300

112

110

109

111   141   113   111

FIG. 5

112

110

111

300

VIII

113   109

-141-

VIII

3

FIG. 6

FIG. 7